# NEW EUROPEAN PATENT SPECIFICATION

(11) **EP 3 638 375 B2**
(45) Date of publication and mention of the opposition decision: **11.03.2026**
(45) Mention of the grant of the patent: 22.06.2022
(21) Application number: 18730718.6
(22) Date of filing: 07.06.2018
(51) Int. Cl.: A61Q 5/12, A61Q 5/02, A61K 8/42, A61K 8/34, A61K 8/365, A61K 8/362, A61K 8/36, A61K 8/81

(54) **HAIR COMPOSITION HAVING IMPROVED RINSING PROPERTIES**
HAARZUSAMMENSETZUNG MIT VERBESSERTEN SPÜLEIGENSCHAFTEN
COMPOSITION CAPILLAIRE PRÉSENTANT DE MEILLEURES PROPRIÉTÉS DE RINÇAGE

(30) Priority: 15.06.2017 EP 17176249
(43) Date of publication of application: 22.04.2020
(73) Proprietor: Unilever IP Holdings B.V., 6708 WH Wageningen (NL); Unilever Global IP Limited, Wirral, Merseyside CH62 4ZD (GB)
(72) Inventor: COAN, Lynsey, Joanne, Bebington Wirral Merseyside CH63 3JW (GB); GILES, Colin, Christopher, David, Bebington Wirral Merseyside CH63 3JW (GB); GLENDAY, Jennifer, Amy, Sheffield S8 8TD (GB); GUTIERREZ-ABAD, Raquel, Bebington Wirral Merseyside CH63 3JW (GB); LUCK, Matias, Wirral Merseyside CH62 2EY (GB)
(74) Representative: Chisem, Janet
(86) International application number: PCT/EP2018/064955
(87) International publication number: WO 2018/228898

(56) References cited:
- WO-A1-2006/010440
- WO-A1-2009/153281
- WO-A1-2009/153281
- WO-A2-99/34768
- US-A1- 2016 374 924

## Description

### Field of the Invention

The invention is concerned with compositions as defined in the appended claims, that are formulated as conditioners for the treatment of hair (typically after shampooing) and subsequent rinsing and particularly relates to an improved rinse-off hair treatment composition that enables less water to be used during use.

### Background and Prior art

Reducing the amount of water that is used in everyday tasks and activities, such as washing of hair and bodies, reduces the energy required to process and deliver it to homes, businesses and communities. This in turn helps to reduce pollution and conserve fuel resources. Reducing the amount of water that goes to waste at home helps protect the wildlife that lives in rivers and wetlands. Thus, hair treatment products that require reduced rinsing to remove from the hair not only save consumers time and effort but can also save ecosystems, energy and water.

Technologies are known that improve rinsing properties of products from hair or hands. WO 13/092708 (L'Oreal) discloses a cosmetic composition, especially a hair composition, comprising at least one anionic or nonionic associative polymer, at least one fixing polymer and at least one specifically defined nonionic surfactant. A styling gel conforming to this composition is purported to have improved hold of the hairstyle over time and to be easily removed from the hands and the hair with water, without shampoo or soap.

WO 15/001071 (L'Oreal) discloses a non-colouring hair composition comprising 2 - 60 wt % of at least one anionic copolymer, water-soluble inorganic salts and one or more alkaline agents. The anionic copolymer may be an anionic associative polymer, such as are capable of associating reversibly with one another or with other molecules. Numerous benefits of these compositions are disclosed, and they are said to be particularly capable of generating an adequate foam, in terms of quality and quantity, and of giving the hair satisfactory cosmetic properties, such as sheen, softness, smoothness, disentangling and suppleness, most particularly on dry hair. Further they are purported to be rinsed out faster than conventional shampoos, to have a more pronounced treating nature and to give the hair more lightness, in particular wet hair. They don't necessarily require the addition of viscosity enhancers, can afford varied textures and are better tolerated by the scalp and the eyes. Detergent compositions used on hair which are said to provide a foaming effect and good cosmetic properties, are exemplified.

Related case WO15/001072 (L'Oreal) discloses a self-foaming non-colouring hair composition comprising from 2 to 60 wt % of one or more anionic, or non-ionic associative polymers, surfactant and propellant gas.

WO 09/153281 (Unilever) discloses a hair conditioning composition comprising hydrophobically modified anionic polymer. The inclusion of a crystalline wax as a structurant is preferred and exemplified. The polymer is said to provide better rinse-off properties and an example discloses better ease of rinse in a composition comprising the polymer compared with a similar composition without the polymer.

Related case WO 09/153280 (Unilever) discloses hair conditioning compositions comprising hydrophobically modified anionic polymer, a silicone and a fatty acid to give improved deposition of silicone. Better rinse off properties are mentioned but silicone deposition is exemplified.

US 2016/0374924 (The Procter & Gamble Company) discloses a method of preparing a hair conditioning composition, wherein the composition comprises by weight: (a) from about 0.1% to about 8% of a cationic surfactant comprising a mono-alkyl amine cationic surfactant; (b) from about 1% to about 15% of a high melting point fatty compound; (c) from about 0.05% to about 6% of an anionic polymer comprising a vinyl monomer (A) with a carboxyl group, wherein the vinyl monomer (A) is contained in the polymer at a level of from about 40 mass % to about 100mass % based on the total mass of the anionic polymer; (d) from about 0.5% to about 20% of a polyol having a molecular weight of from about 40 to about 500; and (e) an aqueous carrier; wherein the method comprises the steps: mixing the cationic surfactant, high melting point fatty compound, polyol and aqueous carrier to form an emulsion; and adding the anionic polymer to the emulsion before, during or after forming the emulsion. A hair conditioning composition made by this method is also disclosed.

Compositions that are easy to rinse do not necessarily require less water to accomplish the rinse. They may, for example, require less mechanical agitation, or even no agitation, but a longer rinse time, so less effort is required but not less water. Despite the technological advances there remains a need for conditioning compositions for use on hair, that require reduced water to rinse effectively and quickly without compromising the performance of the product as a conditioner.

We have now surprisingly found that a conditioning composition that comprises a conditioning base and a hydrophobically modified anionic polymer can be used in the treatment of hair to reduce the amount of water required to rinse without reducing the conditioning benefits on the hair. We have found that when a consumer rinses conditioner from his/her hair, he/she will stop rinsing when a satisfactory constant level of smooth feel is reached (referred to herein as the "rinsed friction plateau"). The composition of the invention enables the consumer to reach his/her rinsed friction plateau sooner, thus causing him/her to stop rinsing and therefore consume less water.

### Definition of the Invention

Accordingly, there is provided a hair treatment composition, see the appended claims for the definition.

Disclosed is a use of a hydrophobically modified anionic polymer in a hair treatment composition of the first aspect, to save water during a rinse step of a conditioning process.

The amount of water required to rinse the composition form hair is reduced by the method disclosed. Thus less water is required when using a composition of the invention. Therefore, water is saved.

The method of the invention preferably saves water during the rinse step of conditioning process.

In the method of the invention, preferably, the hair is rinsed with water until a constant friction is reached.

The use of the invention saves water without compromising on the performance of the product, in terms of wet conditioning and dry conditioning.

The use of the invention, is preferably to save water during a rinse step of a conditioning process where the hair is rinsed until a constant friction is reached.

### The hydrophobically modified anionic polymer

Preferably, the hydrophobically modified polymer is an acrylate or methacrylate polymer. Preferably, the hydrophobic modification comprises alkylation. Preferably, the alkyl group comprises from 6 to 30 carbons, more preferably from C12 to C30, even more preferably from 16 to 28 and most preferably from 18 to 24 carbons.

A preferred polymer is sold by Rohm & Haas under the tradename Aculyn, the most preferred of which is Aculyn 28^{™}.

The polymer is present at from 0.01 to 5 wt %, more preferably from 0.02 to 0 5 wt %, even more preferably from 0.03 to 4 wt % and most preferably from 0.05 to 4 wt %, by total weight of the hair treatment composition.

### The conditioning base

Compositions in accordance with the invention are preferably formulated as conditioners for the treatment of hair (typically after shampooing) and subsequent rinsing.

The conditioning base forms a gel phase.

The conditioning base comprises a combination of i) an amidoamine having from 10 to 22 carbon atoms, and ii) a water soluble non-amphiphilic acid.

Preferably, the amidoamine corresponds to the general formula (I): 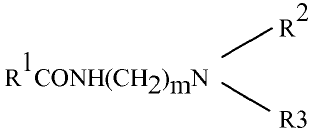
in which R¹ is a hydrocarbyl chain having 10 to 22, preferably 16 to 22 carbon atoms,
R² and R³ are independently selected from hydrocarbyl chains of from 1 to 10 carbon atoms, and
m is an integer from 1 to about 10; and

As used herein, the term hydrocarbyl chain means an alkyl or alkenyl chain.

Preferred amidoamine compounds are those corresponding to formula (I) in which
R¹ is a hydrocarbyl residue having from about 16 to about 22 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine (TAS), stearamidoethyldiethylamine, and mixtures thereof, most preferably stearamidopropyldimethylamine.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid (ii) may be an organic or mineral water soluble non-amphiphilic acid, preferably organic, which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of lactic acid, acetic acid, tartaric acid, fumaric acid, and mixtures thereof, most preferably lactic acid.

The primary role of the water soluble non-amphiphilic acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the water soluble non-amphiphilic acid is included in a sufficient amount to protonate all the amidoamine present, i.e. at a level which is at least equimolar to the amount of amidoamine present in the composition.

### The fatty alcohol

The compositions of the invention comprise a fatty alcohol having a carbon-carbon chain length of from C₈ to C₂₂.

The combined use of fatty alcohols and cationic surfactants in conditioning compositions is preferred because this leads to the formation of a lamellar phase, in which the cationic surfactant is dispersed.

The fatty alcohol comprises from 8 to 22 carbon atoms, preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions for use in the invention.

The level of fatty alcohol in conditioners for use in the invention will generally range from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### Other materials

### Cationic surfactants

Conditioner compositions will preferably comprise one or more other cationic conditioning surfactants which are cosmetically acceptable and suitable for topical application to the hair.

Preferably, the cationic conditioning surfactants have the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic conditioning surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

In conditioners for use in the invention, the level of other cationic conditioning surfactant will generally range from 0.01 to 10%, more preferably 0.05 to 7.5%, most preferably 0.1 to 5% by total weight of cationic conditioning surfactant based on the total weight of the composition.

### Silicones

Compositions according to the invention, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer. A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐOH)₂

wherein the mean value of a is 2 or more and the mean value of b is 6 or more.

Mixtures of any of the above described silicone emulsions may also be used.

The above described silicone emulsions will generally be present in a composition of the invention at levels of from 0.05 to 10%, preferably 0.05 to 5%, more preferably from 0.5 to 2% by total weight of silicone based on the total weight of the composition.

### Further Ingredients

The composition according to the invention may comprise any of a number of ingredients which are common to conditioning compositions

Other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. (ii) hair fibre benefit agents. Examples are:
- ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
- free fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

In a second aspect there is provided a method for the manufacture of a conditioning composition according to the first aspect. The method comprising forming a conditioning gel phase which comprises a cationic surfactant and a fatty material and, separately forming a solution of the hydrophobically modified polymer, optionally with a cationic surfactant, which, if present, is added to the water first.

The two mixtures are then added to one another before the remaining ingredients are added to form the conditioning composition.

Preferably, the extra ingredients include perfumes, thickeners, preservatives, colours and conditioning silicones.

### Example 1: Composition 1 in accordance with the invention and Comparative Composition A

The following compositions were prepared:

**Table 1: Compositions of Comparative A and Composition 1 in accordance with the invention**

| Material (based on 100 % active) | Amount (wt %) | |
|---|---|---|
| | Comparative A | Composition 1 |
| Behenyl Trimethyl Ammonium Chloride | 0.7 | 0.7 |
| Stearamidopropyldimethylamine | 0.7 | 0.7 |
| Lactic acid | 0.35 | 0.35 |
| Ceterearyl Alcohol | 4 | 4 |
| Amodimethicone/Dimethicone | 1.5 | 1.5 |
| Acrylates/Beheneth-25 Methacrylate Coploymer (Aculyn 28) | 0 | 0.1 |
| Fragrance/preservatives/water | to 100 | to 100 |

The conditioners A and 1 were prepared using the following method:
1. Water was added to a suitable vessel, lactic acid and the copolymer were added, and the vessel heated to 80 °C.
2. Cetearyl alcohol was then added to the formulation along with tertiary amine salt (stearamidopropyldimethylamine).
3. At 80°C the Behenyl Trimethyl Ammonium Chloride (BTAC) was added and the resultant mixture mixed until opaque and thick.
4. The heat was then turned off and the quench water was added.
5. The mixture was then cooled to below 40°C the rest of the materials, including fragrance, were added.
6. Finally the formulation was mixed at high shear on a Silverson mixer at 5000rpm for 5 minutes.

### Example 2: Treatment of hair with compositions A and 1

The hair used was dark brown European hair, in switches of 5 g weight and 6 inch length.

Hair was first treated with a cleansing shampoo using the following method:-

The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

The wet hair was then treated with Conditioner A or 1 using the following method:-Conditioner was applied to the wet hair at a dose of 0.2 ml of conditioner per 1g of hair and massaged into the hair for 1 minute. The hair was rinsed under running water for 1 minute and excess water removed.

### Example 3: Friction measurement of hair treated with Compositions A and 1

Friction was measured using the apparatus and method of the invention as follows:
Friction was measured using a TA.XT2i Texture Analyser supplied by Stable Micro Systems, Surrey, UK, and a friction probe in the form of a stainless steel cylinder, which was coated with rubber material and fitted with a weight. The friction probe had surfactant on its outer (contact) surface. The load on the friction contact was approximately 138 g. When in use, an area of contact between the outer surface of the friction probe and the hair of approximately 1.0 cm² was achieved.

Surfactant was added to the probe as follows:
The probe was first washed with an aqueous composition of Sodium Lauryl Ether Sulphate (SLES) at a concentration of 14 wt %, by weight of the total aqueous surfactant composition, and rinsed with water. The probe was then soaked in a dilute solution of SLES having a concentration of 14 ppm, for 2 minutes, and then dried for 2 hours.

The methodology used to assess the friction properties of hair treated with Conditioners A and 1 was as follows: A switch of hair was securely mounted onto the texture analyser, the hair fibres being aligned by combing before being secured in a flat configuration. The hair was immersed in the water bath. The friction probe was placed onto the hair and moved along the hair at a speed of 10 mms⁻¹ to measure the friction between the probe and the hair. The measurement was repeated 30 times.

The friction values reported below are of friction hysteresis in units of gfmm, obtained by integrating the total measured friction force over the total distance travelled by the probe, with and against cuticle.

The conditioner was pre-diluted at 20, 40 , 80 and 160 parts water and used to treat hair using the method described above. Friction measurements were then performed.

Immersed friction measured on hair switches treated with Conditioners A and 1 are given in the Table below.

### Example 4: Friction measurement of hair treated with Compositions A and 1

**Table 2: Friction (gfmm) of hair treated with Comparative A and Composition 1 in accordance with the invention, after dilution levels of 20, 40, 80 and 160.**

| Dilution | Comparative A | Composition 1 | significance |
|---|---|---|---|
| 20 | 102 | 93 | ns |
| 40 | 86.7 | 111.9 | >95% |
| 80 | 92.2 | 128.3 | >95% |
| 160 | 153.7 | 124 | Ns |

It will be seen that a rinsed friction plateau is reached for hair treated with the composition of the invention sooner than with the comparative composition (at a dilution of 80 for composition 1 compared with 160 for composition A).

### Example 5: Comparative Compositions B and C. representative of the prior art

The following compositions were prepared:-
Compositions B and C are representative of the prior art; as given in Table 3 below

**Table 3: Composition of Comparative Compositions B and C**

| Material (based on 100 % active) | Amount (wt %) | |
|---|---|---|
| | Comparative B | Comparative C |
| Behenyl Trimethyl Ammonium Chloride | 1.05 | 1.05 |
| Stearamidopropyldimethylamine | 1.50 | 1.50 |
| Cetearyl Alcohol | 6.0 | 6.0 |
| Acrylates/Beheneth-25 Methacrylate Coploymer (Aculyn 28) | 0.10 | 0.10 |
| Stearic acid | 0.1 | - |
| Paraffin wax | 1.0 | - |
| Conditioning silicone | 3.0 | 3.0 |
| Fragrance | 1.0 | 1.0 |
| Disodium EDTA | 0.10 | 0.10 |
| Phenoxyethanol | 0.40 | 0.40 |
| Methylparaben | 0.20 | 0.20 |
| Water | To 100 | To 100 |

The conditioners B and C were prepared using the following method:
- Approximately 35 % wt. of the water was heated to 65 to 70°C prior to addition of stearmidopropyl dimethylamine with mixing until completely dissolved.
- The polymer, Aculyn 28, was then added at high shear.
- Separately, behenyltrimethyl chloride, cetearyl alcohol, stearic acid (and paraffin if present) were melted together and the resultant molten mixture added to the aqueous phase.
- The rest of the water was added as mixture was cooled to 40 to 45°C before the conditioning silicones, fragrances and preservatives were added.

### Example 6: Viscosity of Compositions B and C under dilution

When a conditioning gel phase composition is applied to hair during a wash/care process, the gel phase is deposited onto the hair surface. When the deposited gel phase comes into contact with water (during a rinse step), the structure of the gel phase must be broken up in order for it to be efficiently removed from the hair. The greater the disruption to the gel phase, the easier and faster it is removed and, *ipso facto,* the less water is required to complete the rinse. The disruption to the composition gel phase can be indicated by a reduction in its viscosity upon dilution with water.

For any given quantity of water, the extent of viscosity reduction indicates how quickly and easily it will be removed from the hair. This correlates with the amount of water used to rinse a conditioning composition from hair.

In the following examples, viscosity measurements were carried out on aqueous dilutions of the neat composition (Compositions 1, B and C).

Samples were measured using a Brookfield viscometer with a T-A spindle as well as RV5.

The samples were prepared as 150 g dilutions as follows:
Composition (for example 75 g for a 1 in 2 dilution) was added to a beaker. Water (75 g for a 1 in 2 dilution) was then added in small amounts with mixing until homogeneous.

The sample was left to equilibrate for one hour before measurement with the Brookfield viscometer.

In this way, a series of dilutions were prepared (ensuring consistent mixing and speed of water addition throughout).

The samples were measured using the Brookfield RVDV-II+ viscometer with the following conditions: T-A bar spindle: 0.5rpm; 60s measurement; 5 replicates per sample.

The results are given in the following table:

**Table 4: Viscosity of Composition 1 (in accordance with the invention) and Comparative Compositions B and B (representative of the prior art).**

| | **Viscosity / cP** | | | | **Normalised data** | | |
|---|---|---|---|---|---|---|---|
| **Dilution** | **1** | **B** | **C** | | **1** | **B** | **C** |
| Neat | 628000 | 781600 | 628000 | | 500000 | 500000 | 500000 |
| 1 in 1.25 | 159200 | 260000 | 197600 | | 126751.6 | 166325.5 | 157324.8 |
| 1 in 1.5 | 82400 | 175200 | 193600 | | 65605.1 | 112077.8 | 154140.1 |
| 1 in 1.75 | 47200 | 143200 | 156800 | | 37579.62 | 91606.96 | 124840.8 |
| 1 in 2 | 21600 | 97600 | 144800 | | 17197.45 | 62436.03 | 115286.6 |
| 1 in 3 | 1600 | 17600 | 58400 | | 1273.885 | 11258.96 | 46496.82 |
| 1 in 4 | 800 | 5600 | 15200 | | 636.9427 | 3582.395 | 12101.91 |

It will be seen that the viscosity of the composition in accordance with the invention drops dramatically faster than that of the prior art examples, ensuring saving of water during a rinse step of a conditioning process.

## Claims

1. A hair treatment composition comprising
a) a conditioning base comprising
i) an amidoamine having from 10 to 22 carbon atoms,
ii) a water soluble non-amphiphilic acid, selected from the group consisting of lactic acid, acetic acid, tartaric acid, fumaric acid, and mixtures thereof;
iii) a fatty alcohol having from 8 to 22 carbon atoms;
b) from 0.01 to 5 percent wt. of a hydrophobically modified anionic polymer, comprising hydrophobic modification that comprises an alkyl group comprising from 12 to 30 carbon atoms; and
c) water.

2. Composition according to claim 1 wherein the polymer is an acrylate polymer.

3. Composition according to any preceding claim wherein the polymer is a methacrylate polymer.

4. Composition according to any preceding claim wherein the amidoamine is selected from stearamidopropyldimethylamine, stearamidoethyldiethylamine, and mixtures thereof.

5. Composition according to any preceding claim that comprises an other cationic conditioning surfactant in an amount of from 0.01 to 10 percent.

6. A method of saving water during a conditioning process, comprising the steps of applying to hair a composition comprising
a) a conditioning base comprising
i) an amidoamine having from 10 to 22 carbon atoms, and
ii) a water soluble non-amphiphilic acid, selected from the group consisting of lactic acid, acetic acid, tartaric acid, fumaric acid, and mixtures thereof;
iii) fatty alcohol having from 8 to 22 carbon atoms,
b) from 0.01 to 5 percent wt. of a hydrophobically modified anionic polymer comprising hydrophobic modification that comprises an alkyl group comprising from 12 to 30 carbon atoms; and
c) water; and rinsing the hair with water, compared with a similar composition that does not comprise a hydrophobically modified anionic polymer comprising hydrophobic modification that comprises an alkyl group comprising from 12 to 30 carbon atoms.

7. A method as claimed in claim 6 wherein the composition is as defined in any one of claims 2 to 5.

8. A method of claim 7, wherein the hair is rinsed with water until a constant friction is reached.

## Patentansprüche

1. Haarbehandlungszusammensetzung, umfassend
a) eine Konditionierungsgrundlage, umfassend
i) ein Amidoamin mit 10 bis 22 Kohlenstoffatomen,
ii) eine wasserlösliche nicht-amphiphile Säure, ausgewählt aus der Gruppe, bestehend aus Milchsäure, Essigsäure, Weinsäure, Fumarsäure und Mischungen davon;
iii) einen Fettalkohol mit 8 bis 22 Kohlenstoffatomen;
b) 0,01 bis 5 Gew.-% eines hydrophob modifizierten anionischen Polymers, umfassend eine hydrophobe Modifikation, die eine Alkylgruppe umfasst, umfassend 12 bis 30 Kohlenstoffatome; und
c) Wasser.

2. Zusammensetzung nach Anspruch 1, wobei das Polymer ein Acrylatpolymer ist.

3. Zusammensetzung nach einem vorhergehenden Anspruch, wobei das Polymer ein Methacrylatpolymer ist.

4. Zusammensetzung nach einem vorhergehenden Anspruch, wobei das Amidoamin aus Stearamidopropyldimethylamin, Stearamidoethyldiethylamin und Mischungen davon ausgewählt ist.

5. Zusammensetzung nach einem vorhergehenden Anspruch, die ein anderes kationisches konditionierendes Tensid in einer Menge von 0,01 bis 10% umfasst.

6. Verfahren zum Einsparen von Wasser während eines Konditionierungsprozesses, umfassend die Schritte des Auftragens einer Zusammensetzung auf das Haar, umfassend
a) eine Konditionierungsgrundlage, umfassend
i) ein Amidoamin mit 10 bis 22 Kohlenstoffatomen und
ii) eine wasserlösliche nicht-amphiphile Säure, ausgewählt aus der Gruppe, bestehend aus Milchsäure, Essigsäure, Weinsäure, Fumarsäure und Mischungen davon;
iii) Fettalkohol mit 8 bis 22 Kohlenstoffatomen;
b) 0,01 bis 5 Gew.-% eines hydrophob modifizierten anionischen Polymers, umfassend hydrophobe Modifikation, die eine Alkylgruppe umfasst, umfassend 12 bis 30 Kohlenstoffatome; und
c) Wasser; und
Spülen des Haars mit Wasser,
verglichen mit einer ähnlichen Zusammensetzung, die kein hydrophob modifiziertes anionisches Polymer enthält, umfassend eine hydrophobe Modifikation, die eine Alkylgruppe umfasst, umfassend 12 bis 30 Kohlenstoffatome.

7. Verfahren, wie im Anspruch 6 beansprucht, wobei die Zusammensetzung wie in irgendeinem der Ansprüche 2 bis 5 definiert ist.

8. Verfahren nach Anspruch 7, wobei das Haar mit Wasser gespült wird, bis eine konstante Reibung erreicht ist.

## Revendications

1. Composition pour le traitement de cheveux comprenant
a) une base de conditionnement comprenant
i) une amidoamine ayant de 10 à 22 atomes de carbone,
ii) un acide non amphiphile soluble dans l'eau, choisi dans le groupe consistant en l'acide lactique, l'acide acétique, l'acide tartrique, l'acide fumarique, et les mélanges de ceux-ci;
iii) un alcool gras ayant de 8 à 22 atomes de carbone;
b) de 0,01 à 5 % en poids d'un polymère anionique hydrophobiquement modifié, comprenant une modification hydrophobe qui comprend un groupe alkyle comprenant de 12 à 30 atomes de carbone; et
c) de l'eau.

2. Composition selon la revendication 1, dans laquelle le polymère est un polymère d'acrylate.

3. Composition selon l'une quelconque des revendications précédentes, dans laquelle le polymère est un polymère de méthacrylate.

4. Composition selon l'une quelconque des revendications précédentes, dans laquelle l'amidoamine est choisie parmi la stéaramidopropyldiméthylamine, la stéaramidoéthyldiéthylamine, et les mélanges de celles-ci.

5. Composition selon l'une quelconque des revendications précédentes, qui comprend un autre tensioactif de conditionnement cationique dans une quantité de 0,01 à 10 pourcents.

6. Procédé d'économie d'eau pendant un procédé de conditionnement, comprenant les étapes d'application aux cheveux d'une composition comprenant
a) une base de conditionnement comprenant
i) une amidoamine ayant de 10 à 22 atomes de carbone, et
ii) un acide non amphiphile soluble dans l'eau, choisi dans le groupe consistant en l'acide lactique, l'acide acétique, l'acide tartrique, l'acide fumarique, et les mélanges de ceux-ci;
iii) un alcool gras ayant de 8 à 22 atomes de carbone;
b) de 0,01 à 5 % en poids d'un polymère anionique hydrophobiquement modifié, comprenant une modification hydrophobe qui comprend un groupe alkyle comprenant de 12 à 30 atomes de carbone; et
c) de l'eau; et de rinçage des cheveux avec de l'eau, en comparaison avec une composition similaire qui ne comprend pas un polymère anionique hydrophobiquement modifié comprenant une modification hydrophobe qui comprend un groupe alkyle comprenant de 12 à 30 atomes de carbone.

7. Procédé selon la revendication 6, dans lequel la composition est comme définie dans l'une quelconque des revendications 2 à 5.

8. Procédé selon la revendication 7, dans lequel les cheveux sont rincés avec de l'eau jusqu'à ce qu'une friction constante soit atteinte.
